# FASCICULE DE BREVET EUROPEEN

(11) **EP 0 746 254 B1**
(45) Date de publication et mention de la délivrance du brevet: **29.08.2001**
(21) Numéro de dépôt: 95900167.8
(22) Date de dépôt: 27.10.1994
(51) Int. Cl.: A61B 17/58

(54) **INSTRUMENTATION D'OSTEOSYNTHESE RACHIDIENNE A ELEMENTS DE LIAISON MULTIPLES**
INSTRUMENTATION FÜR DIE WIRBELSÄULENPOSTEOSYNTHESE MIT MEHREREN VERBINDUNGSELEMENTEN
SPINAL OSTEOSYNTHESIS DEVICE HAVING MULTIPLE LINKING MEMBERS

(43) Date de publication de la demande: 11.12.1996
(73) Titulaire: Biomet S.A., 13170 Les Pennes Mirabeau (FR)
(72) Inventeur: GUILLERMAIN, Pierre, F-13012 Marseille (FR); PAGAZANI, Gerard, F-13630 Eyragues (FR); REYNIER, Yves, F-13009 Marseille (FR); CASTERA, Gerard, F-13008 Marseille (FR); CREPIN, Bruno, F-13570 Barbentane (FR); PY, Jean-Pierre, F-13160 Chateaurenard (FR); DUBOIS, Gilles, F-31400 Toulouse (FR)
(74) Mandataire: Roman, Michel
(86) Numéro de dépôt international: FR9401252
(87) Numéro de publication internationale: WO9613219

(56) Documents cités:
- EP-A- 0 301 489
- FR-A- 2 615 095
- FR-A- 2 657 775
- FR-A- 2 709 057
- US-A- 4 836 196

## Description

La présente invention a pour objet une instrumentation d'ostéosynthèse rachidienne à éléments de liaison multiples.

Elle se rapporte au domaine chirurgical de la réduction et de la fixation des déplacements du rachis dorsal et lombaire d'origine traumatiques, dégénératifs ou tumoraux.

Les interventions d'ostéosynthèse sur la colonne vertébrale exigent un matériel instrumental très élaboré et très spécifique. De nombreuses recherches ont été effectuées dans ce domaine et ont donné lieu au dépôt de toute une série de brevets.

Les systèmes les plus utilisés aujourd'hui sont basés sur l'utilisation d'implants pédiculaires vissables dont les têtes sont réunies par des éléments de liaison extrêmement variés pouvant consister en plaques, tiges, ligaments, crochets ou autres, le plus souvent associés à des dispositifs d'articulation plus ou moins complexes.

Pour être en mesure de traiter tous les cas pouvant se présenter, le praticien doit généralement disposer d'un nombre très important de composants différents, provenant fréquemment de plusieurs fabricants et ne pouvant de se fait pas être combinés. Il est évident que cet état de chose grève les coûts opératoires.

Il faut cependant signaler l'existence du brevet N° FR 2 615 095 concernant une instrumentation pour la correction de scolioses lombaires par voie postérieure comportant, pour chaque vertèbre, une plaque transversale de maintien traversée par deux vis pédiculaires à double filetage maintenant deux tiges associées solidarisées auxdites vis par des pinces.

Les plaques de maintien transversales, nécessitées par cette instrumentation pour obtenir une prise vertébrale solide permettant d'agir sur la vertèbre dans toutes les directions, entraînent un surcoût de fabrication et des contraintes limitant le champs d'application de cette instrumentation.

Le brevet N° FR 2 657 775 décrit un système comportant des vis pédiculaires pouvant être reliées soit par des plaques, soit par des tiges, grâce à des pièces intermédiaires relativement peu nombreuses. Toutefois, avec ce matériel, les plaques ne sont utilisables que pour autant que les vis pédiculaires à relier sont parallèles entre elles, ce qui est rarement le cas en pratique.

Signalons en outre qu'aucun de ces matériels ne permet l'emploi de ligaments.

Le système selon la présente invention supprime tous ces inconvénients. En effet, il permet avec le même implant pédiculaire, pouvant être vissée au plus proche du centre de rotation de la vertèbre d'assurer tous les types de correction par rapport à l'organe de liaison des différents implants, cet implant pouvant utiliser indifféremment des plaques, tiges ou ligaments, la mise en place de chaque implant s'effectuant indépendamment de leur élément de liaison, ce qui assure un maximum de facilité pour leur implantation dans les pédicules vertébraux, et une souplesse d'utilisation rendant possible, avec un nombre limité d'éléments, de faire face à toutes les situations.

Il est constitué d'un implant pédiculaire dont la partie inférieure est pourvue d'un filetage avec extrémité auto-taraudeuse permettant son vissage dans le rachis, et la partie supérieure comporte une collerette tronconique et une partie filetée destinée à recevoir un écrou à une embase conique permettant de fixer aussi bien des plaques perforées ou des tiges de liaison que des cages à ligaments.

Sur les dessins annexés, donnés à titre d'exemples non limitatifs de formes de réalisation de l'objet de l'invention:
la figure 1 est une vue postérieure d'un élément de rachis dans lequel ont été implantés des implants pédiculaires selon l'invention, reliés d'un côté par une plaque, et de l'autre par une tige.
la figure 2 montre une plaque perforée vue en plan, en coupe longitudinale et en coupa transversale,
la figure 3 représente une tige de liaison avec son dispositif de fixation,
la figure 4 est une coupe suivant les flèches A-A de la figure précédente,
la figure 5 montre un implant pédiculaire en élévation,
la figure 6 représente l'écrou de serrage en plan et coupe axiale,
la figure 7 représente dans les mêmes conditions un lingot permettant une jonction entre plaque et implant pédiculaire en dépit d'une inclinaison de ce dernier,
la figure 8 est une vue de dessus de la partie femelle de la cage à ligaments
et la figure 9 est une coupe suivant les flèches B-B de la figure 8 représentant les parties femelle et mâle, non assemblées, de la cage à ligaments.

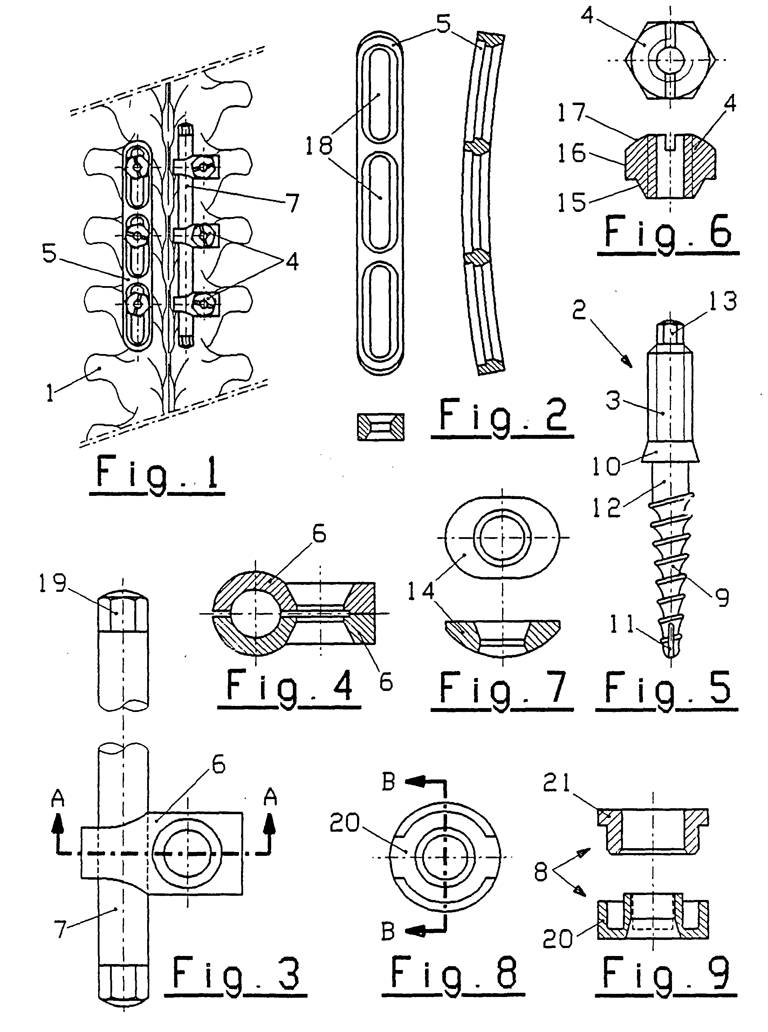

Le système, figures 1 à 9, est constitué d'un implant pédiculaire 2 vissable dans le rachis 1 et comportant une partie filetée 3 destinée à recevoir un écrou 4 de serrage permettant de fixer soit des plaques perforées 5, soit des étriers doubles 6 supportant des tiges 7, soit des cages à ligaments 8.

Cet implant unique, de par sa conformation, permet la mise en traction-détraction des différents segments vertébraux entre eux ainsi que les dérotations par mouvements imprimés aux organes de liaison liant les divers implants. Il est composé de trois parties :

Une portion inférieure pédiculo-corporéale. La portion corporéale 9, tronconique, comporte un filetage de type cortico-spongieux à pas large avec une collerette 10 à visée rétentive et une extrémité auto-taraudeuse 11. Le filetage est échelonné sur deux tiers de sa longueur. Le tiers restant correspondant à la portion pédiculaire 12 est non fileté, du diamètre externe du dernier pas de vis et peut être couvert d'hydroxiapatite. Cette portion dans le pédicule vertébral se termine par une collerette 10 tronconique destinée à recevoir les pièces de liaison qu'elles soient cage à ligament 8, plaque 5 à glissement ou étrier double 6 de conjonction avec les tiges 7. Cette partie tronc conique s'adaptera à la face inférieure des divers éléments précités permettant, lorsque cela est nécessaire notamment sur les montages sur tiges ou plaques, la mise en traction ou détraction des différents implants les uns par rapport aux autres. Le profilé particulier de la zone pédiculo-corporéale permet une transmission des forces au long de l'implant vissé amenant des possibilités à visée de réduction transmises au niveau de la jonction pédiculo-corporéale, soit au niveau du mur postérieur vertébral.

La partie supérieure de l'implant est constituée de deux portions, une portion distale 13 permettant la préhension de l'implant par un instrument spécifique pour le visser dans la vertèbre, et une portion proximale 3 naissant de la collerette 10, portion filetée, destiné à recevoir l'écrou de serrage 4 terminal.

En fonction de l'organe de liaison choisi, s'il s'agit de la plaque 5, le système de glissement permettant la mise en traction ou détraction est assuré par la présence d'un lingot 14 de forme ovalaire permettant en serrage sub-total d'assurer un phénomène de glissement permettant la mise en traction ou détraction des diverses pièces osseuses les unes par rapport aux autres. Ce lingot est conçu pour permettre éventuellement une jonction entre la vis et la plaque en dépit d'une inclinaison de l'implant de l'ordre de 40° par rapport à l'axe de la plaque. Ainsi, la configuration particulière de la charnière lombo-sacrée ne pose pas de problème mécanique susceptible de provoquer de rupture de matériel. Si l'organe de liaison choisi est une tige 7, un système de double étrier 6 biconcave permet d'enserrer la tige, la liaison avec implant et l'embase tronconique permettant les mêmes phénomènes de détraction-traction.

L'écrou 4 est composé de trois portions, une embase conique 15 qui vient épouser la rainure supérieure soit de la plaque 5, soit du lingot 14, soit du double étrier 6 préhenseur de tige 7 permettant de refermer le système de glissement en morthèse qui réalise l'originalité du système, une portion hexagonale 16 permettant sa préhension afin de pouvoir le serrer, une portion supérieure 17 arrondie visant à masquer l'extrémité la plus distale de l'implant vissé afin de le rendre non vulnérant pour les parties molles.

L'embase conique 15 de l'écrou, associée avec la collerette 10 également conique de l'implant pédiculaire 2, assure un serrage biconique empêchant tout desserrage et rendant inutile les divers système de freins utilisés habituellement.

Il existe quatre longueurs de plaques 5, pouvant comporter de deux à cinq perforations oblongues 18. Ces perforations, par leur forme allongée, permettent une adaptation facile à tous les écarts inter-pédiculaires et une mise en position aisée.

Ces plaques sont pré-courbées en lordoses. Elles sont indéformables.

Les tiges 7 sont prévues en huit longueurs allant de 60 à 210 mm (6 mm de diamètre). Elle comportent à chacune de leur extrémité un embout hexagonal 19 permettant d'effectuer leur dérotation.

Ces tiges sont malléables permettant une adaptation à toutes les déformations rachidiennes ainsi qu'une correction des cyphoses et des scolioses.

Les cages à ligaments 8 sont constituées d'une bague femelle 20 inférieure recevant et d'une bague mâle 21 supérieure pénétrant dans la précédente de manière à assurer un serrage périphérique énergique emprisonnant et bloquant lesdits ligaments.

Il s'agit donc d'un système à glissement permettant la mise en traction-détraction des divers implants entre eux pouvant être reliés indifféremment par des ligaments, des plaques 5 à glissement pourvues de perforations oblongues 18 ou des tiges 7.

Pour certaines applications particulières, le matériel décrit peut être complété par des barres de liaison permettant le montage en cadre, par des crochets sus et sous-lamaires classiques ou encore par des crochets pédiculo-transversaires monoblocs, donnant ainsi au système d'instrumentation rachidienne des possibilités d'adaptation complètes répondant à tous les cas de figure trouvés en pathologie rachidienne. Ce matériel simple, versatile est servi par un ancillaire réduit au maximum, ce qui en fait un matériel universel, d'utilisation simple et dont les expérimentations bio-mécaniques ont montré la fiabilité.

Le positionnement des divers éléments constitutifs donne à l'objet de l'invention un maximum d'effets utiles qui n'avaient pas été, à ce jour, obtenus par des dispositifs similaires.

## Revendications

1. Dispositif d'instrumentation d'ostéosynthèse rachidienne ayant pour objet la réduction et la fixation des déplacements du rachis dorsal et lombaire d'origine traumatiques, dégénératifs ou tumoraux, comprenant d'une part des éléments de liaison multiples constitués notamment de plaques perforées (5), étriers doubles (6) supportant des tiges (7) ou cages à ligaments (8) et, d'autre part, des implants pédiculaire (2) dont la partie inférieure est formée d'un filetage (9) vissable dans le rachis (1), et la partie supérieure comporte une partie filetée (3) destinée à recevoir un écrou (4) de serrage à embase conique (15) permettant la fixation desdits éléments de liaisons en assurant un serrage rendant inutile l'utilisation de freins,
**caractérisé en ce que** chaque implant pédiculaire (2) comprend une portion inférieure pédiculo-corporéale formée d'une partie corporéale (9) à filetage de type cortico-spongieux à pas large échelonné sur deux tiers de la longueur, avec extrémité auto-taraudeuse (11), et d'une partie pédiculaire (12) non filetée se terminant par une collerette (10) tronconique s'évasant vers la partie corporéale, destinée à recevoir les pièces de liaison et s'adaptant à la face inférieure des divers éléments (5, 6, 8) précités, et une partie supérieure constituée elle-même de deux portions, une portion distale (13) permettant la préhension de l'implant par un instrument spécifique pour le visser dans la vertèbre, et une portion proximale (3) filetée naissant de la collerette (10), destiné à recevoir l'écrou de serrage (4).

2. Dispositif selon la revendication 1, se caractérisant par le fait que l'écrou (4) est composé de trois portions, une embase conique (15), une portion hexagonale (16) permettant sa préhension afin de pouvoir le serrer, une portion supérieure (17) arrondie visant à masquer l'extrémité la plus distale de l'implant pédiculaire (2) afin de le rendre non vulnérant pour les parties molles.

3. Dispositif selon l'une quelconque des revendications précédentes, se caractérisant par le fait que les plaques perforées (5) comportent de deux à cinq perforations oblongues (18) et que leur fixation des est assurée au moyen d'un lingot (14) de forme ovalaire permettant en serrage sub-total d'assurer un phénomène de glissement permettant la mise en traction ou détraction des diverses pièces osseuses les unes par rapport aux autres, ce lingot étant conçu pour permettre une jonction entre l'implant pédiculaire (2) et la plaque en dépit d'une inclinaison de l'implant de l'ordre de 40° par rapport à l'axe de la plaque.

4. Dispositif selon la revendication 3, se caractérisant par le fait que les plaques perforées (5) sont pré-courbées en lordose et qu'elles sont indéformables.

5. Dispositif selon la revendication 1, se caractérisant par le fait que les tiges (7) sont maintenues sur l'implant pédiculaire (2) au moyen d'un double étrier (6) biconcave permettant la mise en traction ou détraction des diverses pièces osseuses les unes par rapport aux autres.

6. Dispositif selon la revendication 1, se caractérisant par le fait que les cages à ligaments (8) sont constituées d'une bague femelle (20) inférieure recevant et d'une bague mâle (21) supérieure pénétrant dans la précédente de manière à assurer un serrage périphérique énergique emprisonnant et bloquant lesdits ligaments.

## Patentansprüche

1. Geräteanordnung für Rückgrat-Osteosynthese zum Zweck der Reduzierung und der Fixierung von Verschiebungen der Wirbelsäule traumatischen, degenerativen oder tumoralen Ursprungs im Rücken- und Lendenbereich, mit einerseits mehrfachen Verbindungselementen, im wesentlichen bestehend aus durchbrochenen Stegen (5), Doppel-Klemmen (6) zum Halten von Stiften (7) oder Ligamentkäfigen (8), und andererseits Pedikular-lmplantaten (2), deren unterer Teil durch ein in den Wirbel (1) einschraubbares Gewinde (9) gebildet wird und deren oberer Teil einen Gewindebolzen (3) aufweist, dazu bestimmt, eine Spannmutter (4) mit konischem Ansatz (15) aufzunehmen, und damit die Befestigung der besagten Verbindungselemente erlaubt und eine Spannung gewährleistet, welche die Verwendung von Sicherungen überflüssig macht,
**dadurch gekennzeichnet**, daß jedes Pedikular-Implantat (2) einen unteren, pedikular-korporealen Teil aufweist, gebildet aus einem korporealen Abschnitt (9) mit Gewinde des kortikal-spongiösen Typs, mit breitem sich über zwei Drittel seiner Länge erstreckenden Gang und selbstschneidender Spitze (11), und aus einem pedikularen Abschnitt (12) ohne Gewinde, der in einem stumpfkegeligen, nach dem korporealen Abschnitt sich erweiternden Kragen (10) endet, dazu bestimmt, die Verbindungsteile aufzunehmen und sich an die untere Fläche der verschiedenen vorgenannten Elemente (5, 6, 8) anzupassen, sowie einen oberen Teil aufweist, wiederum bestehend aus zwei Abschnitten, einem distalen Abschnitt (13), welcher das Greifen des Implantats mit einem spezifischen Instrument erlaubt, um es in den Wirbel zu schrauben, und einem proximalen Abschnitt (3) mit Gewinde, der am Kragen (10) seinen Anfang nimmt, dazu bestimmt, die Spannmutter (4) aufzunehmen.

2. Geräteanordnung gemaß Anspruch 1, **dadurch gekennzeichnet**, daß die Mutter (4) aus drei Teilen gesteht, einem konischen Ansatz (15), einem Sechskant (16) zum Greifen zum Zweck ihres Anziehens und einem oberen, abgerundeten Teil (17), dazu bestimmt, die distalste Extremität des Pedikular-Implantats (2) zu verdecken, damit dieses die weichen Teile nicht verletzen kann.

3. Geräteanordnung gemäß irgendeinem der vorausgehenden Ansprüche, **dadurch gekennzeichnet**, daß die durchbrochenen Stege (5) zwei bis fünf längliche Aussparungen (18) aufweisen, und daß ihre Befestigung mittels eines ovalförmigen Blocks (14) erfolgt, der erlaubt, bei einem sub-totalen Anziehen ein Gleit-Phänomen zu gewährleisten, das die Spannung oder Entspannung der verschiedenen Knochenteile untereinander erlaubt, wobei dieser Block dazu ausgelegt ist, eine Verbindung zwischen Pedikular-Implantat (2) und Steg trotz einer Neigung des Implantats in der Größenordnung von 40° in Bezug auf die Achse des Stegs zu ermöglichen.

4. Geräteanordnung gemäß Anspruch 3, **dadurch gekennzeichnet**, daß die durchbrochenen Stege (5) bei der Herstellung wirbelsäulen-gerecht gebogen werden und unverformbar sind.

5. Geräteanordnung gemäß Anspruch 1, **dadurch gekennzeichnet**, daß die Stifte (7) im Pedikular-Implantat (2) mit Hilfe einer bikonkaven Doppel-Klemme (6) gehalten werden, welche die Spannung bzw. die Entspannung der verschiedenen einzelnen Knochenteile untereinander ermöglicht.

6. Geräteanordnung gemäß Anspruch 1, **dadurch gekennzeichnet**, daß die Ligamentkäfige (8), aus einer unteren Aufnahmebuchse (20) und einer in diese eingreifenden Einsatzbuchse (21) besteht und somit eine kräftige peripherische Fixierung gewährleistet, wodurch die besagten Ligamente eingeschlossen und blockiert werden.

## Claims

1. Rachidian osteosynthesis instrumentation device for reducing and fixing spinal and lumbosacral displacements of traumatic, generative or tumourous origin comprising on the one hand multiple liaison elements in particular consisting of perforated plates (5), double stirrups (6) supporting rods (7) or ligament cages, (8) and on the other, pedicular implants (2), the lower part of which consists of a thread (9) which may be screwed in the spine (1), and the upper part comprising a threaded part (3) designed to receive a tightening nut (4) with conical base (15) for fixing the said liaison elements by tightening to make the use of locking systems unnecessary,
**characterised in that** each pedicular implant (2) includes a lower pedico-corporeal section formed of a corporeal part (9) with cortico-spongeous type thread with wide pitch spread over two-thirds of the length, with self-tapping end (11), and unthreaded pedicular part (12) ending in a conical collar (10) with the flared end towards the corporeal part, destined to receive the liaison elements and adapting to the lower face of the various above-mentioned components (5, 6, 8), and an upper part itself consisting of 2 sections, a distal section (13) for gripping the implant using the specific instrument in order to screw it into the vertebra, and a threaded proximal section (3) originating from the collar (10), designed to receive the tightening nut (4).

2. Device according to claim one, **characterised in that** the nut (4) consists of three sections, a conical base (15), a hexagonal section (16) which service to grip the device in order to tighten it, and a rounded upper section (17) designed to hide the end which is the most remote from the pedicular implant (2) and make it less wounding for the soft parts.

3. Device according to any one of the aforesaid claims, **characterised in that** the perforated plates (5) have two to five oblong perforations (18) and that they are fixed by an ingot (14) with oval form for sub-total tightening to allow sliding to take place in order to tension or relieve the various bone elements relative to one another, this ingot being designed to allow joining between the pedicular implant (2) and the plate despite a slope of the implant of around 40° relative to the plate axis.

4. Device according to claim 3 characterised by the fact that the perforated plates (5) are lordosis precurved and that they are indeformable.

5. Device according to claim one, **characterised in that** the rods (7) are held on the pedicular implant (2) by a biconcave double stirrup (6) used to tension or relax various bone sections relative to one another.

6. Device according to claim one, **characterised in that** ligament cages (8) consist of a lower socket ring (20) and an upper insert ring (21) penetrating the former in order to ensure strong peripheral tightening imprisoning and blocking the said ligaments.
